Europäisches Patentamt

European Patent Office

Office européen des brevets

(11) Publication number: **0 485 961 A1**

# EUROPEAN PATENT APPLICATION

(21) Application number: **91119289.6**

(22) Date of filing: **12.11.91**

(51) Int. Cl.5: **A61K 37/02**

(30) Priority: **13.11.90 US 612361**

(43) Date of publication of application:
**20.05.92 Bulletin 92/21**

(84) Designated Contracting States:
**AT BE CH DE DK ES FR GB GR IT LI LU NL SE**

(71) Applicant: **Bristol-Myers Company**
**345 Park Avenue**
**New York New York 10154(US)**

(72) Inventor: **Grove, Robert I.**
**6405 235th Place Southwest**
**Montlake Terrace, WA 98043(US)**
Inventor: **Mazzucco, Charles E.**
**38 Peat Meadow Road**
**New Haven, CT 06513(US)**
Inventor: **Radka, Susan F.**
**909 North 35th Street, Apt. 203**
**Seattle, WA 98103(US)**
Inventor: **Shoyab, Mohammed**
**2405 Westmont Way West**
**Seattle, WA 98199(US)**
Inventor: **Kiener, Peter A.**
**8 Cow Hill Road**
**Killingsworth, CT 06417(US)**

(74) Representative: **Kinzebach, Werner, Dr. et al**
**Patentanwälte Reitstötter, Kinzebach und**
**Partner Sternwartstrasse 4 Postfach 86 06 49**
**W-8000 München 86(DE)**

(54) **Oncostatin M for stimulating LDL uptake and LDL receptor expression.**

(57) The present invention is directed to methods for stimulating low density lipoprotein receptor (LDLR) expression and low density lipoprotein (LDL) uptake. The method of the invention may be useful as a means of reducing the level of plasma cholesterol in an individual, and therefore may be useful in the treatment of hypercholesterolemia and atherosclerosis. In a specific embodiment of the invention, the polypeptide Oncostatin M is used to rapidly stimulate LDL uptake by and LDLR expression in human hepatocytes. A basic mechanism by which Oncostatin M exerts these effects is disclosed. The invention encompasses the use of Oncostatin M, Oncostatin M analogs and Oncostatin M derivatives, whether used alone, in combination, or conjugated to other biologically active molecules or antibodies.

EP 0 485 961 A1

EP 0 485 961 A1

## TABLE OF CONTENTS

1.   Introduction..................................................3

2.   Background of the Invention..................................3

    2.1.   Atherosclerosis......................................3

    2.2.   Role of Low Density Lipoprotein and its
          Receptor in Cholesterol Homeostasis............4

    2.3.   Oncostatin M.........................................5

3.   Summary of the Invention....................................6

4.   Description of the Figures..................................7

5.   Detailed Description of the Invention......................10

    5.1.   Mode of Action by Oncostatin M......................11

    5.2.   Use of Oncostatin M and Oncostatin M
          Analogs to Stimulate LDL Receptor
          Expression.........................................14

    5.3.   Oncostatin M as an Anti-
          Hypercholesterolemia and Anti-
          Artherosclerotic Agent.............................15

6.   Example: Stimulation of LDL Receptor Expression
   in Human Hepatocytes by Oncostatin M.......................16

    6.1.   Materials and Methods...............................16

        6.1.1.   Cells and Reagents.......................16

        6.1.2.   LDL Uptake Assay.........................17

        6.1.3.   LDL Receptor ELISA.......................18

        6.1.4.   DNA Synthesis Assay......................18

        6.1.5.   Sterol Synthesis Asssay..................19

        6.1.6.   Lipid Analysis...........................19

        6.1.7.   Phosphotyrosine Proteins.................20

        6.1.8.   Oncostatin M Receptor
              Crosslinking.............................20

6.2.    Results.......................................21

    6.2.1.    Oncostatin M Stimulates LDL
                Uptake Independent of
                Extracellular LDL Levels..............21

    6.2.2.    LDL Uptake Stimulation by
                Oncostatin M Results from
                Elevated LDLR Expression..............22

    6.2.3.    The Effect of Oncostatin M on
                LDLR Expression Does Not Result
                from Cholesterol Synthesis or
                Cell Growth Stimulation..............22

    6.2.4.    Oncostatin M Binds to a 160 Kd
                Receptor on Hepatocytes and
                Stimulates Tyrosine
                Phosphorylation and
                Diacylglycerol........................23

    6.2.5.    Effect of Oncostatin M on
                Phospholipid Metabolism..............24

## 1. INTRODUCTION

The present invention is directed to methods for stimulating low density lipoprotein receptor (LDLR) expression in hepatocytes, thereby enhancing the cells' capacity to remove low density lipoprotein (LDL) from the extracellular environment, using Oncostatin M and/or other compounds capable of triggering the pathway by which Oncostatin M induces LDLR upregulation. In a particular embodiment of the invention, illustrated by way of example, the stimulating effects of Oncostatin M on LDLR expression and LDL uptake in hepatocytes are described and characterized. The method of the invention may be useful in the treatment of atherosclerosis and hypercholesterolemia.

## 2. BACKGROUND OF THE INVENTION

### 2.1. ARTHEROSCLEROSOSIS

Atherosclerosis is responsible for the majority of myocardial and cerebral infarctions and therefore represents the leading cause of death in industrial nations, including the United States. Studies have demonstrated a clear correlation between the concentration of plasma lipoproteins, principally low density lipoprotein (LDL)/plasma cholesterol, and the incidence of atherosclerosis.

Hypercholesterolemic individuals face an increased risk of developing atherosclerosis and ischemic heart disease. Lowering plasma cholesterol levels in these individuals reduces the risk of atherosclerosis. There is some evidence suggesting that fatty streak atherosclerotic lesions can regress in individuals who decrease their intake of lipids and atherogenic foods. It is possible that some forms of hypercholesterolemia and atherosclerosis may be treatable with agents capable of reducing plasma cholesterol levels.

### 2.2. ROLE OF LOW DENSITY LIPOPROTEIN AND ITS RECEPTOR IN CHOLESTEROL HOMEOSTASIS

The hepatic low density lipoprotein receptor (LDLR) plays a major role in cholesterol homeostasis. Circulating cholesterol in the form of LDL is removed from plasma by the highly specific LDLR and is internalized via receptor-mediated endocytosis. Once internalized, an LDL particle is degraded within the lysosomal compartment, thereby liberating and increasing the intracellular concentration of free cholesterol. The elevated free cholesterol concentration signals the hepatocyte to decrease the rate of transcription of the genes of some of the key enzymes in the cholesterol biosynthetic pathway, resulting in a decrease in de novo cholesterol synthesis. LDLR mRNA and protein are also downregulated by elevated intracellular cholesterol, resulting in decreased ability of the liver to remove additional LDL cholesterol from the plasma. A mechanism to independently upregulate the LDLR would therefore be expected to yield a greater decrease in plasma cholesterol levels.

Mononuclear leukocytes secrete a number of polypeptides which modulate a wide variety of different cellular functions, including proliferation and differentiation. Recently, evidence that macrophages may affect cholesterol homeostasis has emerged. Colony stimulating factors which activate macrophages (M-CSF, GM-CSF) cause a dramatic decrease in total serum cholesterol in humans and in primates. A study in diet-induced hypercholesterolemic rats indicated that injection of a macrophage activator (zymosan) caused a significant decrease in total serum cholesterol. More recent evidence suggested that endotoxin-stimulated macrophage conditioned medium was able to induce a significant increase in LDL uptake and LDLR number in a human liver cell line. The conditioned medium also caused a decrease in cholesterol synthesis following the LDLR effect. This latter finding suggested the possibility that a factor produced by stimulated macrophages acted selectively to upregulate the LDLR.

One of the cell-modulating factors produced by macrophages is the growth regulatory polypeptide Oncostatin M. Originally discovered in conditioned medium from differentiated U937 cells (a macrophage cell line), Oncostatin M is recognized by a high affinity cell surface receptor of approximately 150 Kd. When its effect on LDLR function was investigated, applicants found that the factor increased LDL uptake. There is some evidence suggesting that the LDLR may be regulated by cholesterol-independent mechanisms. Induction of LDLR message by a protein kinase C-stimulator in HepG2 cells and skin fibroblasts has been reported. However, ligands which bind to selective receptors on liver cell membranes and induce the expression of the LDLR by increasing kinase activities have not been identified.

4

## 2.3. ONCOSTATIN M

Oncostatin M, originally identified for its inhibitory effects on human tumor cell lines, was first isolated from phorbol 12-myristate 13-acetate (PMA)-induced human histiocytic lymphoma cells (Zarling et al., 1986, Proc. Natl. Acad. Sci. USA 83: 9739-9743) and from activated T lymphocytes (Brown et al., 1987, J. Immunol. 139: 2977-2983). The molecule is a heat and acid stable protein comprised of a single polypeptide chain of $M_r$ = 28,000. Like other naturally occurring growth regulators, Oncostatin M exhibits a variety of biological activities. Growth inhibition is observed with some, but not all, human tumor cell lines. In contrast, the growth of some normal fibroblasts, such as human foreskin fibroblasts or WI-38 cells, is stimulated by exposure to Oncostatin M (Zarling et al., 1986, Proc. Natl. Acad. Sci. USA 83: 9739-9743).

The gene for Oncostatin M has been cloned and sequenced, and an active form of recombinant Oncostatin M has recently been expressed in mammalian cells (European application EP-A-88 107 180) which is incorporated herein by reference in its entirety). The mature form of Oncostatin M is a glycoprotein containing 228 amino acids, five of which are cysteine residues. The protein has an extremely hydrophilic carboxy terminal domain. Although Oncostatin M is not structurally related to other known cytokines, its mRNA contains an AU-rich region at its 3' untranslated end. This region in the Oncostatin M message is homologous to that of many cytokines, lymphokines and other growth-regulatory molecules, suggesting a common mode of regulating gene expression. A cellular receptor for Oncostatin M has been found on a variety of mammalian cells. The major Oncostatin M receptor molecule is a specific protein of Mr = 150,000-160,000 (Linsley et al, 1989, J. Biol. Chem. 264: 6528-6532).

## 3. SUMMARY OF THE INVENTION

The invention relates to methods of stimulating LDL receptor expression in and LDL uptake by cells of the liver (hepatocytes). In this way, the liver's capacity for removing circulating cholesterol may be significantly enhanced. The method of the invention may be particularly useful in the treatment of hypercholesterolemia, a condition characterized by abnormally high levels of serum cholesterol, and in the treatment of the related disease atherosclerosis.

The invention is based in part on the discovery that Oncostatin M binds to a specific receptor on hepatocytes and triggers a series of molecular events which result in the stimulation of LDL receptor expression. Oncostatin M-stimulated hepatocytes therefore acquire an enhanced ability to remove extracellular LDL/cholesterol. Applicants have identified several of the molecular events which result in the stimulation of hepatocyte LDL receptor expression and LDL uptake capacity, and thus the method of the invention encompasses the use of factors which mimic the stimulation pathway induced by the Oncostatin M polypeptide. Conjugate molecules in which Oncostatin M is covalently coupled to a biologically active molecule, such as a growth factor, cytokine, hormone or antibody, are also utilized in the methods of the present invention. The molecules of the invention can be administered dissolved or dispersed in a carrier, or encapsulated in a liposome or microcapsule.

The invention is described by way of examples demonstrating the effects that Oncostatin M has on LDL receptor expression, LDL uptake, intracellular tyrosine kinase activity, phospholipid metabolism, and cholesterol synthesis in the cultured hepatoma cell line HepG2.

## 4. DESCRIPTION OF THE FIGURES

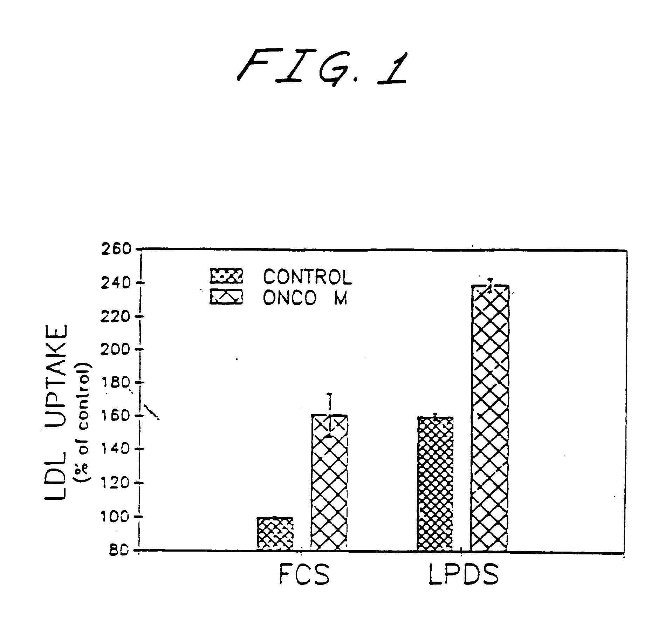

FIG. 1. Stimulation of HepG2 LDL Uptake by Oncostatin M. Oncostatin M (100 ng/ml) was added to monolayers of HepG2 cells cultured in the presence of FCS (with cholesterol) or LPDS (without cholesterol) and incubated for 18 hours. LDL uptake was assayed as described in Section 6.1.2., *infra*. Control uptake was 150 ng LDL/mg and 245 ng LDL/mg cell protein for cells in FCS and in LPDs, respectively. The data represent the means and SEM for 5 different experiments.

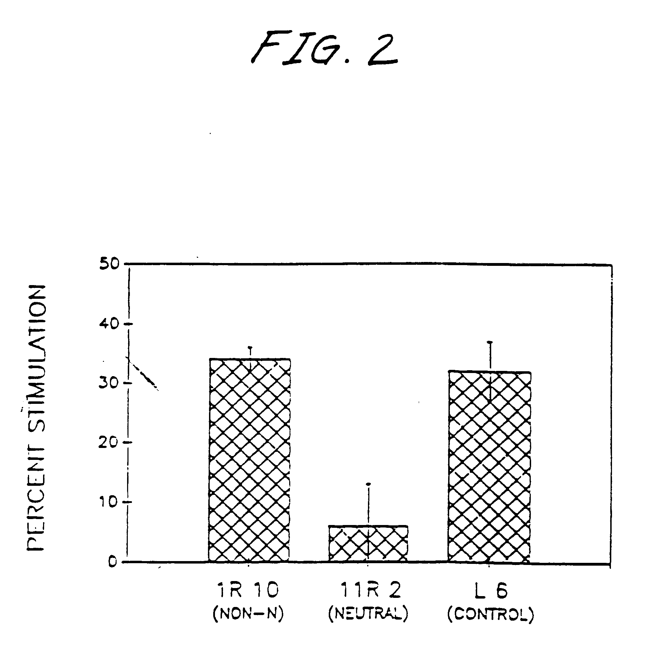

FIG. 2. Inhibition of Oncostatin M Effect by Anti-Oncostatin M Antibody. Cultures of HepG2 cells in LPDs-containing media were treated with 5 ug/ml of anti-Oncostatin M neutralizing antibody (11R2), anti-Oncostatin M non-neutralizing antibody (IR12), or an unrelated antibody (L6). After 15 minutes some of the monolayers were stimulated with 100 ng/ml Oncostatin M. LDL uptake was assayed after 18 hours. The data represent the results of 3 different experiments, each with 6 determinations.

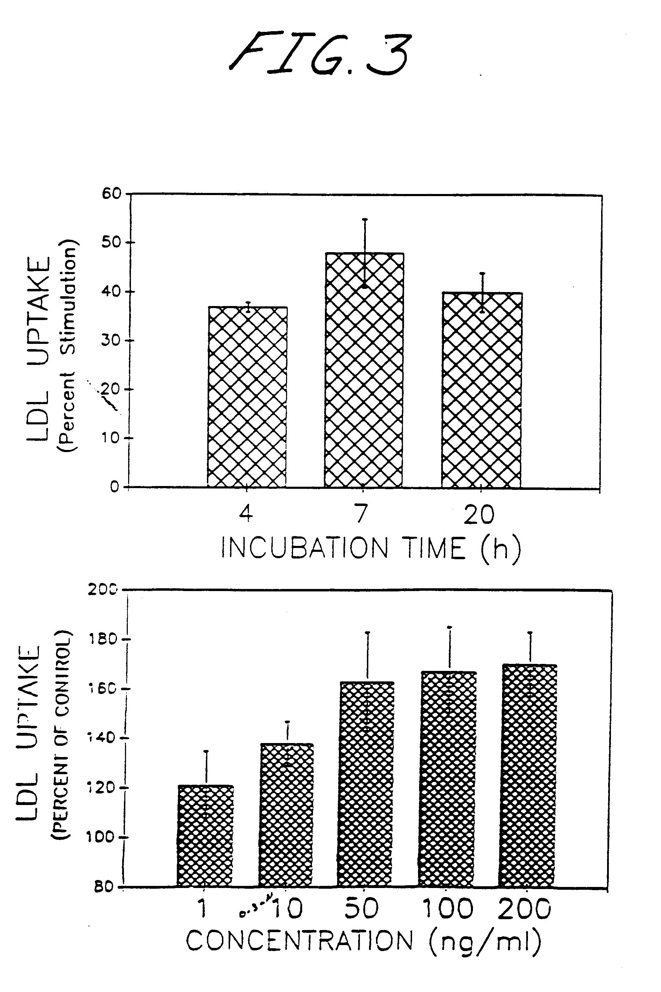

FIG. 3. Oncostatin M Stimulation of LDL Uptake. A: Time Dependence. Oncostatin M (100 ng/ml) was incubated with HepG2 monolayers cultured in medium containing LPDS for the indicated times before assaying for LDL uptake. Addition times were staggered in order to assay LDL uptake simultaneously in all the cultures. The data represent means and SEM for 3 different experiments. B: Concentration Dependence. The indicated concentrations of Oncostatin M were added for a total of 20 hours.

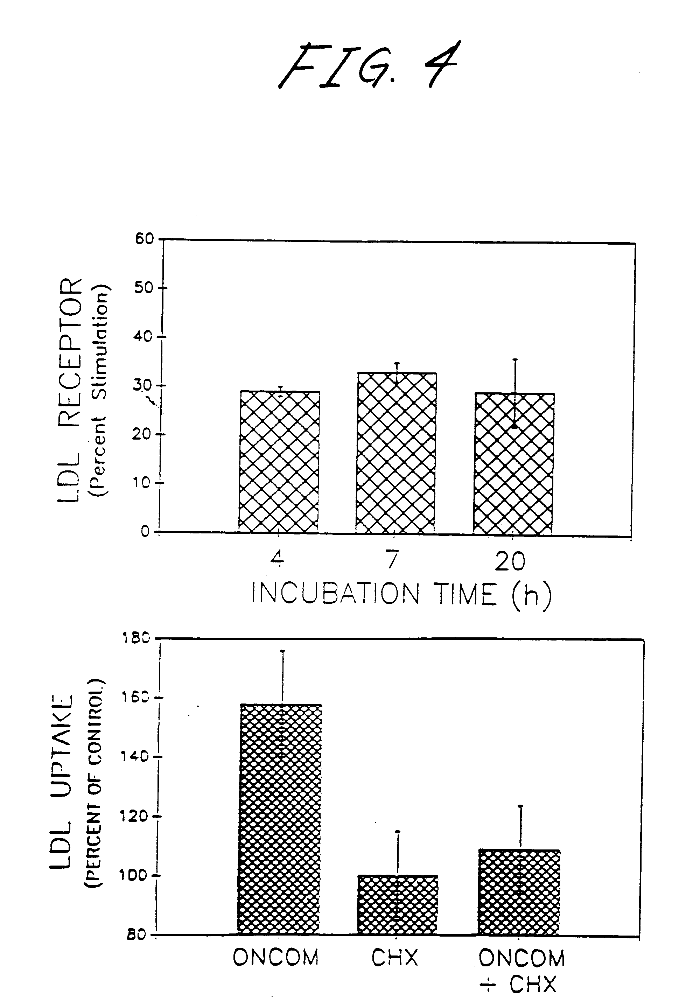

FIG 4. Stimulation of HepG2 LDL Receptors by Oncostatin M and Inhibition With Cycloheximide. A: Surface LDL receptors were quantitated using an ELISA technique in HepG2 monolayers stimulated with Oncostatin M (100 ng/ml) for the indicated times. The ELISA experiments were set up in parallel with those used to obtain the data of FIG. 3A and in order to obtain both receptor number and LDL uptake data from cells treated identically. The data represent the means and SEM from 3 different experiments. B: Cycloheximide (5 ug/ml) was added to HepG2 cultures 30 min before Oncostatin M (100 ng/ml) and LDL uptake was assayed 4 hours later. Cell proteins were determined in the same culture from which the ELISA data were obtained.

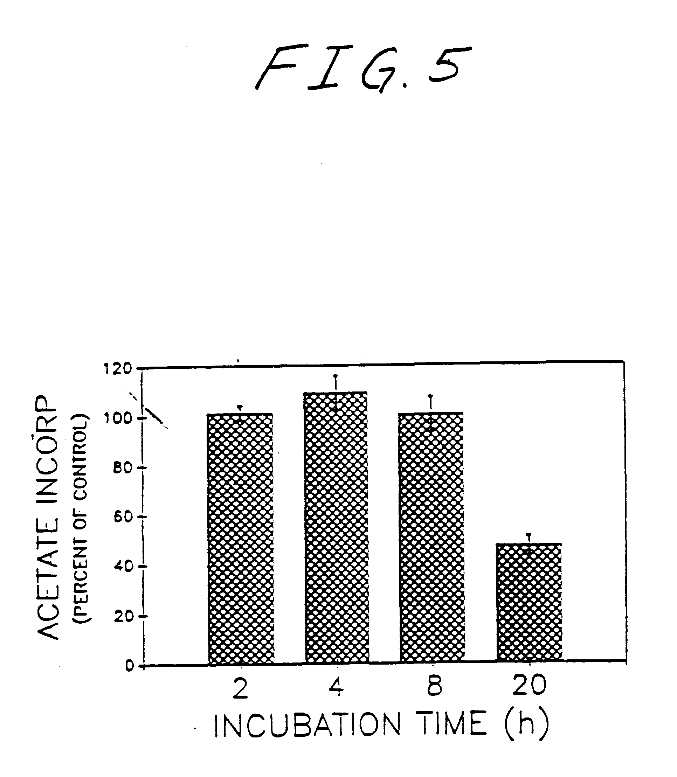

FIG. 5. Effect of Oncostatin M on Sterol Synthesis in HepG2 Cells. Cells incubated in LPDS were treated with Oncostatin M (100 ng/ml) for the indicated times. One hour before the end of the experiment, $[^{14}C]$ acetate (2 uCi/ml) was added and incorporation of radiolabel into non-saponifiable lipids was determined as outlined in Section 6.1.5., *infra*. The data represent the means and SEM for 3 different experiments.

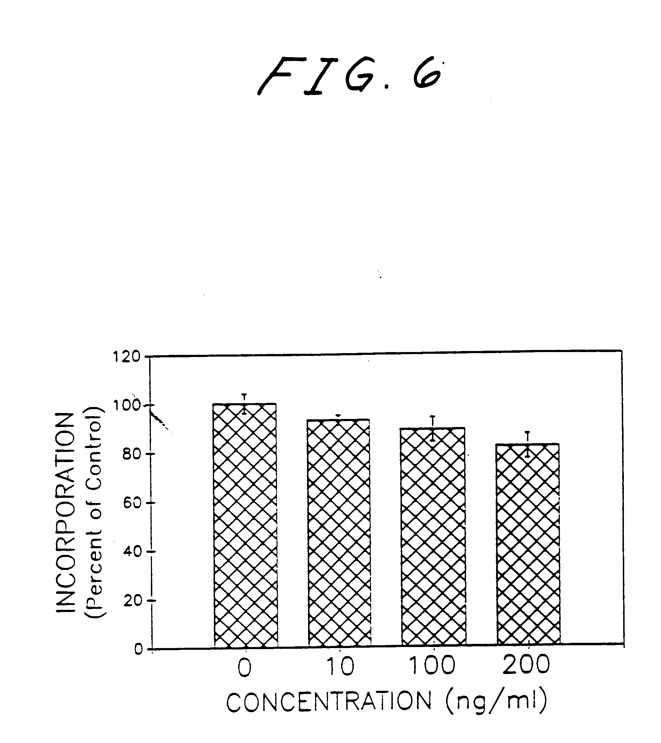

FIG. 6. Effect on Oncostatin M on HepG2 DNA Synthesis. Monolayers were incubated with Oncostatin M (100 ng/ml) for the indicated times. Two hours before the end of the experiment, 2 uCi/ml $[^{3}H]$ thymidine were added. Incorporation of radiolabel into TCA precipitable material was evaluated as described in Section 6.1.4., *infra*.

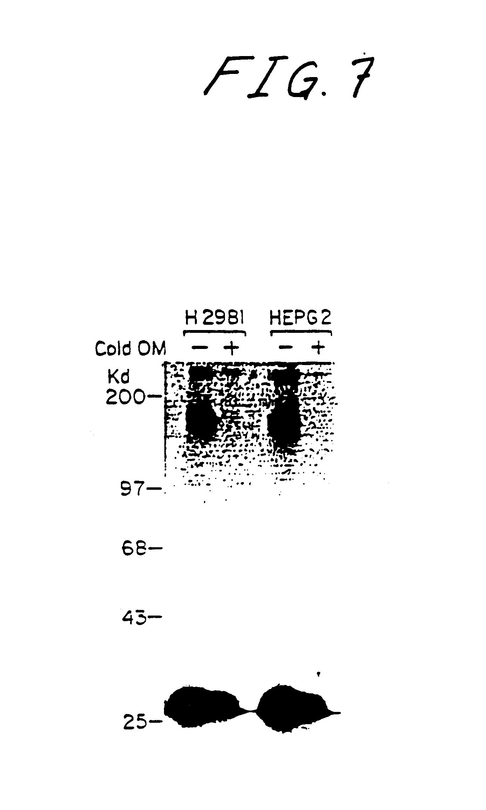

FIG. 7. Crosslinking of $[^{125}I]$-Oncostatin M to a HepG2 Cell Surface Protein and to the H2981 cell Oncostatin M receptor. Procedure as described in Section 6.1.8., *infra*.

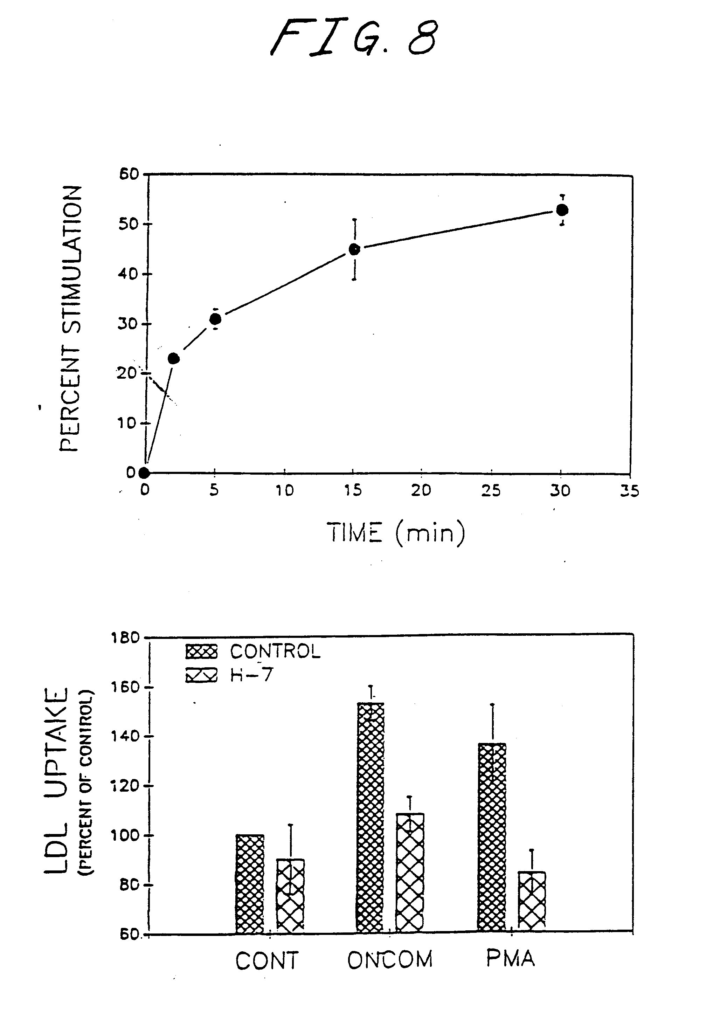

FIG. 8. A: Stimulation of HepG2 Diacylglycerol by Oncostatin M. HepG2 cells were incubated overnight in LPDS medium and then for 5 hours with $[^{3}H]$ glycerol (10 uCi/ml). At the indicated times before the end of the experiment, 100 ng/ml Oncostatin M was added. The monolayers were washed twice to remove free radiolabel and then solubilized with 0.1% sodium dodecyl sulfate. The lipids were extracted and separated as described in Section 6.1.6., *infra*. The data represent means and SEM for 3 different experiments. B: HepG2 cultures were treated with 10 ug/ml H-7 for 30 min and then challenged for 4 hours with Oncostatin M (100 ng/ml) or PMA (10 nM) before LDL uptake was assayed.

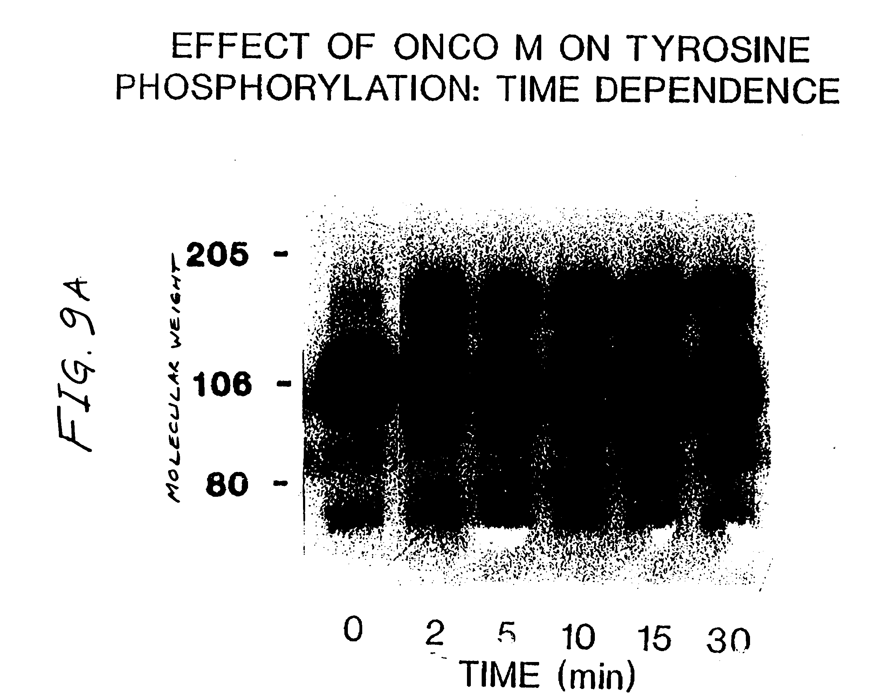

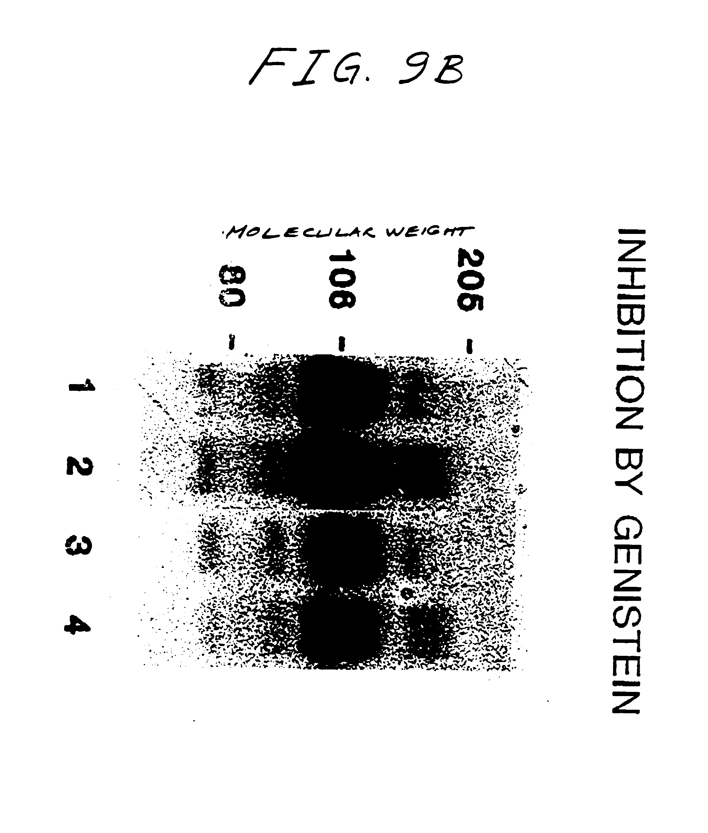

FIG. 9. Effect on Oncostatin M on tyrosine phosphorylation in HepG2 cells. A: Time dependence. HepG2 monolayers were incubated with 100 ng/ml Oncostatin M for the indicated times, washed with phosphate buffered saline containing 1 mM sodium vanadate and then lysed and analyzed for phosphotyrosines as outlined in Section 6.1.7., *infra*. B: Inhibition of the Oncostatin M Effect by Genistein. HepG2 monolayers were incubated with 100 ng/ml Oncostatin M and/or 30 uM/ml genistein as indicated below, and were then lysed and analyzed for phosphotyrosines as outlined above. Lane 1, control cells; lane 2, cells treated with Oncostatin M for 10 minutes; lane 3, cells treated with genistein for 40 minutes; lane 4, cells treated with genistein for 30 minutes, then treated with Oncostatin M for 10 minutes.

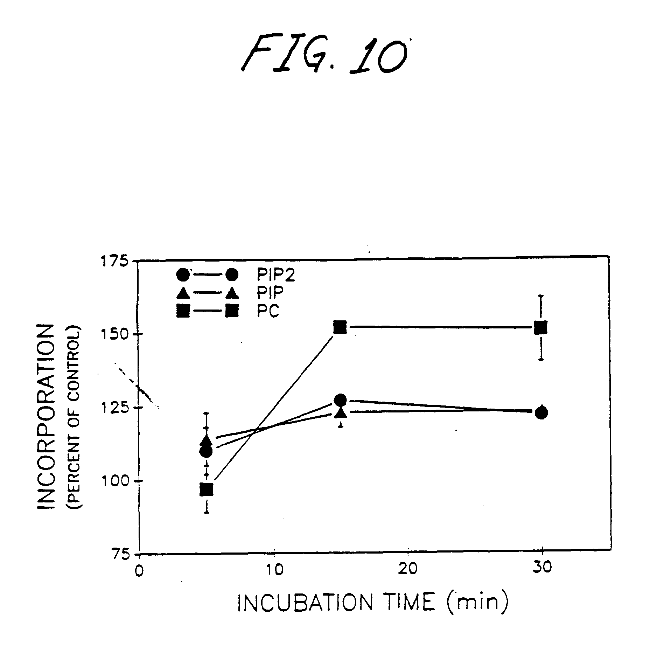

FIG. 10. Alteration of $[^{32}P]$-Incorporation Into HepG2 Phospholipids by Oncostatin M. HepG2 cells were incubated for 16 hours in LPDS and then 50 uCi/ml $[^{32}P]$ were added. Thirty minutes later, the cells were challenged with Oncostatin M for the indicated times. Radiolabel incorporated into HepG2 phospholipids were evaluated as described in Section 6.1.6., *infra*.

## 5. DETAILED DESCRIPTION OF THE INVENTION

The present invention is directed to methods for inducing the expression of LDL receptors (LDLRs) on the surface of hepatocytes, thereby enhancing the cells' ability to remove LDL from the extracellular environment. The method of the invention may be useful as a means of reducing the level of circulating serum LDL/cholesterol in humans. Applicants have discovered that the cytokine Oncostatin M is capable of rapidly inducing the expression of the LDLR in a sterol synthesis-independent system and have identified the basic pathway by which Oncostatin M stimulates LDLR upregulation. The method of the invention involves, generally, triggering this LDLR upregulation pathway such that LDLR cell surface expression is increased. In a particular embodiment, this pathway is triggered by the specific binding of Oncostatin M to a hepatocyte cell surface receptor. The method of the invention is described by way of examples in which the effects of Oncostatin M on LDLR expression, LDL uptake, intracellular tyrosine kinase activity, phospholipid metabolism, and cholesterol synthesis are determined using an *in vitro* system.

Oncostatin M and other compositions capable of triggering this LDLR upregulation pathway may be useful in modulating serum cholesterol levels *in vivo* and, more particularly, may be useful as anti-hypercholesterolemia and/or anti-atherosclerotic agents. The effectiveness of a compound in stimulating LDLR expression may be evaluted directly by quantitating the increased number of LDLRs on the surface of cultured cells in response to the compound using, for example, an assay such as the LDLR ELISA described in Section 6.1.3., *infra*. Alternatively, a compound's effectiveness may be evaluated by measuring the amount of LDL uptake induced by the test compound using, for example, the LDL uptake assay described in Section 6.1.2., *infra*.

## 5.1. MODE OF ACTION BY ONCOSTATIN M

Applicants have identified several basic steps along the pathway by which Oncostatin M stimulates LDLR expression in cultured hepatocytes. Oncostatin M binds to a selective receptor on human liver cells, thereby triggering a tyrosine kinase activity and a dramatic increase in the level of diacylglycerol, most likely resulting from tyrosine kinase-induced phospholipase C activation. The elevation of diacylglycerol in the cell results in the activation of protein kinase C, which in turn induces the upregulation of LDLR expression. It is not known whether the tyrosine kinase activity induced by Oncostatin M acts via messenger pathways unconnected with protein kinase C.

As described more fully in the examples presented in Section 6., et seq., *infra*, Oncostatin M upregulates the LDLR by a cholesterol-independent mechanism. In this regard, the time course studies demonstrating that Oncostatin M-induced LDL uptake and LDLR expression occurs long before inhibition of cholesterol synthesis provide strong evidence that Oncostatin M does not exert its effects by first inhibiting cholesterol synthesis. Oncostatin M also appears to exert its effect independent of the level of cholesterol in the medium. In contrast, agents such as oxidosqualene, which directly inhibit the activity of cholesterol synthetic enzymes such as HMG CoA reductase, decrease cholesterol synthesis before upregulating the LDLR. It is likely that Oncostatin M induces LDLR expression via a mechanism involving intracellular second messengers activated by a Oncostatin M-receptor binding event.

Oncostatin M binds to a 160 Kd receptor on HepG2 cells and induces rapid increases in both tyrosine phosphorylation and diacylglycerol levels (Section 6.2.4., *infra*). Since inhibition of tyrosine kinase activity inhibited both the diacylglycerol and LDLR expression levels induced by Oncostatin M, it appears that tyrosine kinase activity and diacylglycerol are two of the components involved in the mechanism by which Oncostatin M exerts its effect. Furthermore, a connection between tyrosine kinase activity and diacylglycerol stimulation induced by Oncostatin M is supported by results demonstrating that the addition of a phorbol ester to tyrosine kinase-inhibited HepG2 cells restores elevated LDLR expression.

The phospholipid effects of Oncostatin M disclosed in Section 6.2.5., *infra*, are also consistent with a second messenger induction system. Degradation of both phosphoinositides and phoshatidylcholine can yield diacylglycerol, an endogenous protein kinase C (PKC) activator. The observed alterations in phospholipid metabolism and the increase in diacylglycerol suggest that Oncostatin M may be exerting some of its effects via a protein phosphorylation mechanism. This is supported by the indication that the protein kinase C inhibitor H-7 reverses the effect of Oncostatin M, thereby indicating an involvement of PKC and/or other kinases in the Oncostatin M-stimulated LDLR upregulation pathway.

The reason for the delayed onset of cholesterol synthesis inhibition stimulated by Oncostatin M after 8 hours of incubation is not obvious (Section 6.2.3., *infra*). One possible explanation is that LDL cholesterol taken from the medium by the increase in LDLR expression inhibits cholesterol synthesis via the well characterized feedback suppression mechanism. However, the observation that Oncostatin M induces the same magnitude of LDLR expression regardless of the presence or absence of cholesterol in the medium tends to argue against this explanation. Another possibility is that Oncostatin M-stimulated second messengers downregulate cholesterol synthetic enzymes. Since hydroxymethylglutaryl coenzyme A reductase activity appears to be negatively regulated by phosphorylation, LDL uptake together with a phosphorylation component may be involved in the delayed downregulation of cholesterol synthesis by Oncostatin M.

## 5.2. USE OF ONCOSTATIN M AND ONCOSTATIN M ANALOGS TO STIMULATE LDL RECEPTOR EXPRESSION

In accordance with the invention, Oncostatin M may be obtained by techniques well known in the art from a variety of cell sources which synthesize bioactive Oncostatin M including, for example, cells which naturally produce Oncostatin M and cells transfected with recombinant DNA molecules capable of directing the synthesis and/or secretion of Oncostatin M. Alternatively, Oncostatin M may be synthesized by chemical synthetic methods including but not limited to solid phase peptide synthesis. Methods for the production of Oncostatin M are described in copending application Serial No. 144,574 filed January 15, 1988, a continuation-in-part of application Serial No. 046, 846 filed May 4, 1987, a continuation-in-part of application Serial No. 935,283 filed November 26, 1986, a continuation-in-part of application Serial No. 811,235 filed December 20, 1985, each of which is incorporated by reference herein in its entirety.

In the practice of the method of the invention, the use of Oncostatin M obtained by any method, as well as the use of modified or truncated Oncostatin M molecules and Oncostatin M analogs which retain the capacity to induce the induction of LDLR expression, are within the scope of the invention. In this regard, variations in the Oncostatin M primary structure, as well as variations in higher levels of structural organization, the type of covalent bonds linking the amino acid residues, and/or addition of groups to the terminal residues of Oncostatin M are within the scope of the invention. For example, the Oncostatin M molecule used in accordance with the invention may include conservative or non-conservative alterations in the amino acid sequence which result in silent changes that preserve the functionality of the molecule including, for example, deletions, additions and substitutions. Such altered Oncostatin M molecules may be desirable where they provide certain advantages in their use. As used herein, conservative substitutions would involve the substitution of one or more amino acids within the sequence of Oncostatin M with another amino acid having similar polarity and hydrophobicity/hydrophilicity characteristics resulting in a functionally equivalent molecule. Such conservative substitutions include but are not limited to substitutions within the following groups of amino acids: glycine, alanine; valine,isoleucine, leucine; aspartic acid, glutamic acid; asparagine, glutamine; serine, threonine; lysine, arginine; phenylalanine, tyrosine; and methionine, nor-leucine.

In another embodiment of the invention, Oncostatin M may be linked to carrier molecules. For example, Oncostatin M could be covalently coupled to an antibody molecule specific for hepatocytes or for some other cell surface antigen which will allow Oncostatin M to be targeted to cells which express that particular antigen. Similarly, Oncostatin M may be linked to other "targeting" molecules such as hormones, growth factors, cytokines, etc. In this way, the Oncostatin M molecule could be altered so that it is taken up by cells that may not express a receptor for the particular Oncostatin M molecule chosen for use. Such coupling techniques are well known in the art and can include, for example, the use of cross-linking agents, Schiff-base formation, amide bonds, peptide bonds, sulfide bonds, etc.

### 5.3. ONCOSTATIN M AS AN ANTI-HYPERCHOLESTEROLEMIA AND ANTI-ATHEROSCLEROTIC AGENT

Oncostatin M may be useful in the treatment of hypercholesterolemia and atherosclerosis, since it can enhance the capacity of hepatocytes to remove extracellular LDL. This aspect of the invention involves contacting the cells of an individual's liver with Oncostatin M so that the liver's capacity to remove plasma LDL, and thus cholesterol, is enhanced. An amount of Oncostatin M effective at stimulating LDLR expression and LDL uptake may be formulated in suitable pharmacological carriers and administered *in vivo* via any appropriate route including but not limited to injection, infusion and selective catheterization. In addition, Oncostatin M may be linked to a carrier or targeting molecule and/or incorporated into liposomes, microcapsules, and controlled release preparations prior to administration

The ability of Oncostatin M compositions to stimulate LDLR expression and LDL uptake can readily be tested using *in vitro* assay systems such as those described in Section 6 *et seq*. herein. The functional equivalence and/or increased efficacy of modified Oncostatin M molecules or Oncostatin M analogs may be evaluated similarly.

### 6. EXAMPLE: STIMULATION OF LDL RECEPTOR EXPRESSION IN HUMAN HEPATOCYTES BY RECOMBINANT ONCOSTATIN M

### 6.1. MATERIALS AND METHODS

### 6.1.1. CELLS AND REAGENTS

The hepatoma cell line, HepG2, was obtained from ATCC (Bethesda, MD) and cultured in RPMI medium supplemented with 10% fetal bovine serum. The cell culture reagents were obtained from Gibco Laboratories (Grand Island, NY), except for lipoprotein deficient serum and the fluorescent Dil-LDL (Biotechnologies Inc, Boston). Genistein was obtained from (ICN). Oncostatin M and anti-Oncostatin M monoclonal antibodies were provided by Oncogen (Seattle). The OM1 and OM2 monoclonal antibodies are directed against epitopes on native Oncostatin M; OM1 does not neutralize the effects of Oncostatin M in a growth inhibition assay which OM2 completely abbrogates Oncostatin M-induced activity in a concentration-dependent fashion (Radka, et al., manuscript in preparation). Radioisotopes were purchase from New England Nuclear (Boston). All other reagents were from Sigma (St. Louis).

## 6.1.2. LDL UPTAKE ASSAY

LDL uptake in HepG2 cells cultured in 12 well plates (Costar) at $2 \times 10^5$ per well was assayed by the fluorescent LDL method using a fluorescence microscope and video camera (SIT 66) attached to computer (Laser 25) with JAVA image analysis software (Jandel Scientific, CA) as previously reported (Grove, et al.,). This method has been shown to be comparable to [$^{125}$I]-LDL methods and therefore to reliably reflect LDL receptor activity and regulation. Specifically, 2 ug/ml DiI-LDL in serum free media was added to HepG2 monolayers for two hours. The monolayers were washed 3 times to remove free DiI-LDL and fixed with 4% formalin in phosphate-buffered saline. Accumulated fluorescence was measured by quantitating the average intensity of 25 - 50 cells. Three difference fields in each of duplicate or triplicate monolayers were quantitated and averaged. The amount of LDL protein taken up was determined as follows. Unfixed cell monolayers were solubilized with 0.1% sodium dodecyl sulfate, transferred into glass tubes, and the lipids (and DiI) were extracted into chloroform using the Bligh-Dyer extraction procedure. Fluorescence in the chloroform layer was quantitated with a fluorescence spectrophotometer (Perkin-Elmer) and compared with a standard curve generated by extracting DiI from known quantities of DiI-LDL. Coomassie blue kits (Pierce) were used to estimate cell proteins.

## 6.1.3. LDL RECEPTOR ELISA

The amount of surface LDL receptor was estimated with an ELISA which employed the LDL receptor antibody, C-7 (Brown and Goldstein). The HepG2 monolayers were fixed with 4% formalin and blocked for 2 hours with phosphate-buffered saline (PBS) containing 3% (w/v) bovine serum albumin (BSA). The blocking solution was replaced with PBS containing BSA (0.5%) and 4 ug/ml C-7 and incubated at 25%C for 2 hours. The monolayers were extensively washed to remove excess antibody with a wash solution (PBS, 0.5% BSA, 0.05% Triton X-100) and then incubated for 1 hour in a solution containing the second antibody (PBS plus 20 ug/ml of peroxidase-conjugated goat anti-mouse IgG; Cooper Biochemicals). After removal of excess antibody with the wash solution, the colorimetric assay solution consisting of 0.1 M citrate-phosphate (pH 5), O-diphenylene diamine (4 mg/ml; Sigma), and hydrogen peroxide ($1.2 \times 10^{-4}$%) was added to the monolayers. Color development was stopped after 8-10 minutes by the addition of HCl (2.5 N, final concentration). Optical density was measured at 490 nm in a V max microtiter plate reader (Molecular Devices).

## 6.1.4. DNA SYNTHESIS ASSAY

[$^3$H]-thymidine at 1 uCi/ml was incubated with HepG2 monolayers for 1 hour. The monolayers were washed 4 times with phosphate-buffered saline and precipatated with ice-cold 5% trichloroacetic acid. The washed precipitates were solubilized with 0.25% sodium dodecyl sulfate and counted in a scintillation counter.

## 6.1.5. STEROL SYNTHESIS ASSAY

Cholesterol synthesis was estimated from the sterol synthesis method of Tanaka, et al. Briefly, cell cultures which had incubated with 2 uCi/ml [$^{14}$C]-acetate for 1 hour were washed once with PBS and incubated with 1.5 N NaOH for 10 minutes before transfer into glass test tubes. The lysate was saponified at 75°C for 1.5 hours. The unsaponified lipids were extracted with petroleum ether and the upper phase was dried under a stream of $N_2$ at 45°C. The lipids were first resuspended with ethanol:acetone (1:1) and then the sterols were precipitated by the addition of 1% digitonin. The precipitates were washed twice with acetone and counted in a scintillation counter.

### 6.1.6. LIPID ANALYSIS

Lipids from cells radiolabeled with [$^{32}$P]-orthophosphate or with ($^{3}$H)-glycerol were extracted by a modification of the method of Bligh and Dyer. Extracts, dried under a stream of nitrogen, were solublized with 100 ul of chloroform and spotted on thin-layer chromatography (TLC) plates (Silica Gel 60, Merck). The phospholipids were resolved either with a solvent system designed to separate the inositides (propanol: 4.3 M ammonium hydroxide, 65:35 v/v) or with a system designed to separate phosphatidylcholine and phosphatidylethanolamine (chloroform:methanol:acetic acid:water (50:35:8:1, v/v). Neutral lipids were resolved in a tank containing benzene:ethyl acetate (80:20, v/v). The radiolabeled phospholipids were identified as spots on autoradiographs which comigrated with authentic standards (detected with iodine vapors), were scraped into scintillation vials, and were quantitated in a scintillation counter. Neutral lipid standards (diacylglycerol and triacylglycerol) which were added to the [$^{3}$H]- glycerol labeled lipid extracts before chromatography were identified with iodine vapors, scraped into scintillation vials, and analyzed as outlined above.

### 6.1.7. PHOSPHOTYROSINE PROTEINS

HepG2 cells were plated into six well plates at 4 x 10$^{5}$ cells/well. After 48 hours (about 70% confluency), the cells were incubated with Oncostatin M or drugs for the indicated time, rapidly washed once with ice cold PBS, and lysed with 100ul of SDS-sample buffer containing 1mM mercaptoethanol and 500 uM sodium vanadate. The bufer was rapidly mixed over the entire well before being removed and heated to 100°C for 15 minutes. The samples (25 ul) were separated by SDS-PAGE on 10-20% gradient gels and tranfered to Hybond C Extra membranes (Amersham). The membranes were blocked with PBS containing 5% BSA, 1% ovalbumin, and 1 mM orthovanadate for 16 hours at 4°C. Proteins containing phosphotyrosine were detected using an affinity purified rabbit antiphosphotyrosine antibody followed by [$^{125}$I]-labeled protein A (ICN).

### 6.1.8. ONCOSTATIN M RECEPTOR CROSSLINKING

Either H2981 cells (lung adenocarcinoma) or HepG2 cells were plated in 24 well tissue culture plates (Costar) and grown to confluence (approximately 2 x 10$^{5}$ cells/well). After two washes with Dulbecco's Modified Eagles' Medium supplemented with 40 mM BES (pH 6.9), 0.1% BSA, and 10% FCS, the cells were incubated for 3 hours at room temperature with 200 ul volumes of Binding Buffer containing 4 nM of [$^{125}$I]-Oncostatin M, either with or without 400 nM of unlabeled Oncostatin M. For cross linking, a 0.4 mM concentration of the homobifunctional noncleavable crosslinker disuccinyl suberate (DSS) was added and the cells incubated for 20 minutes at room temperature. The monolayers were then washed, and the cells were lysed with 2% SDS electrophoresis sample buffer containing 5% 2-mercaptoethanol, boiled for 5 minutes, and then electrophoresed on 7.5% SDS-PAGE. Gels were dried and autoradiographed on Kodak X-AR5 film.

### 6.2. RESULTS

### 6.2.1. ONCOSTATIN M STIMULATES LDL UPTAKE INDEPENDENT OF EXRACELLULAR LDL LEVELS

The effects of Oncostatin M on LDL uptake in LDL receptor upregulated (lipoprotein-depleted serum: LPDS) or downregulated (medium containing lipoprotein cholesterol) HepG2 cultures were investigated. Compared to the controls, Oncostatin M at 100 ng/ml (4 nM) stimulated LDL uptake approximately 60%, in both downregulated and in upregulated cells (FIG. 1). Oncostatin M-stimulated LDL uptake in downregulated cells (FCS) was virtually equal in magnitude to the stimulation induced by the absence of cholesterol in the medium (LPDS controls). Shown in FIG. 2 are the results of a study in which HepG2 cells were preincubated with a control preimmune IgG (L6), a neutralizing antibody specific for Oncostatin M (OM2), or a non-neutralizing antibody specific for Oncostatin M (OM1). The neutralizing antibody OM2 inhibited the LDL uptake stimulated by Oncostatin M while neither of the other antibodies had a significant effect.

The time and concentration dependencies of the Oncostatin M effect are shown in FIG. 3. The concentration of Oncostatin M giving half maximal increases was approximately 3 ng/ml (0.2 nM) while at concentrations of 100 ng/ml or higher, maximum stimulation (70%) was observed (FIG. 3B). Time course studies indicated that the earliest detectable response occurred by 2 hours. The peak response, at 8 hours, was maintained during over 20 hours of incubation (FIG. 3A).

### 6.2.2. LDL UPTAKE STIMULATION BY ONCOSTATIN M RESULTS FROM ELEVATED LDLR EXPRESSION

To determine whether the LDL uptake stimulation was due to increased LDL receptor number, we employed an LDL receptor ELISA assay using the C-7 antibody. The data presented in FIG. 4A reveals that the factor increased LDL receptor number with a time dependence and magnitude similar to those of the LDL uptake response. The results presented in FIG. 4B show that the protein synthesis inhibitor cyclohexamide completely blocked the LDLR upregulation induced by Oncostatin M, indicating that Oncostatin M acts by increasing the expression of the LDLR.

### 6.2.3. THE EFFECT OF ONCOSTATIN M ON LDLR EXPRESSION DOES NOT RESULT FROM CHOLESTEROL SYNTHESIS INHIBITION OR CELL GROWTH STIMULATION

Upregulation of the LDL receptor surface protein occurs in HepG2 cells cultured in the absence of medium cholesterol or when inhibitors of cholesterogenesis are present. To determine whether Oncostatin M-stimulated effects of LDLR expression is dependent on cholesterol synthesis inhibition, radiolabelled acetate incorporation into sterols in HepG2 cells treated with Oncostatin M was investigated. The data indicate that Oncostatin M dramatically inhibited sterol synthesis (60% inhibition), but not until after 8 hours of incubation (FIG. 5).

Some polypeptide growth regulatory factors increase LDLR expression by stimulating cell proliferation and an increased requirement for cholesterol. In contrast, Oncostatin M did not stimulate, but rather slightly inhibited, radiolabled thymidine incorporation in HepG2 cells (FIG. 6). Inhibition of DNA synthesis reached approximately 25% after 24 - 48 hours of Oncostatin M incubation.

### 6.2.4. ONCOSTATIN M BINDS TO A 160Kd RECEPTOR ON HEPATOCYTES AND STIMULATES TYROSINE PHOSPHORYLATION AND DIACYLGLYCEROL

In order to learn more about the mechanism by which Oncostatin M interacted with HepG2 cells, the possibility of a HepG2 cell surface protein which selectively recognized Oncostatin M was explored. Covalent crosslinking techniques demonstrated that $[^{125}I]$-Oncostatin M associated with a high molecular weight protein of approximately 160 Kd (FIG. 7). Crosslinking of radiolabeled Oncostatin M was inhibited when excess unlabeled Oncostatin M was included (FIG. 7, " + "). A cell line known to possess a high density of Oncostatin M receptors (H2981) was used as a positive control. The data presented in FIG. 7 reveal that the Oncostatin M receptor in HepG2 cells crosslinked as much or more radiolabeled Oncostatin M as did the H2981 Oncostatin M receptor.

As HepG2 cells appeared to possess a specific Oncostatin M cell surface receptor, the effects of Oncostatin M on second messenger pathways was examined. When diacylglycerol levels were measured, a 20% increase was detected within 2 minutes (FIG. 8A). The stimulation reached 60% above control values by 30 minutes and remained elevated for at least 60 minutes. The increase in diacylglycerol suggested the possibility of a protein kinase C-dependent mechanism for Oncostatin M. Therefore, the effects of both a stimulator (PMA) and an inhibitor (H-7) of protein kinase C on HepG2 LDL receptor regulation were studied. Incubation with PMA induced LDL uptake with a similar time dependency compared to Oncostatin M. H-7 prevented the increases in LDL uptake induced by both Oncostatin M and PMA (FIG. 8B).

Oncostatin M induced a rapid (within 2 minutes) and sustained increase in tyrosine phosphorylation in HepG2 proteins with molecular weights of 75, 90 and 120 Kd (FIG. 9), an effect which was mitigated by the tyrosine kinase inhibitor genistein (30 ug/ml) . Genistein also inhibited Oncostatin M-induced elevations in diacylglycerol and LDLR activity. The inhibition of the LDLR expression with genistein was reversed by adding phorbol ester (PMA, 50 nM). In contrast, the protein kinase stimulator vanadate appeared to synergistically enhance tyrosine kinase and LDLR expression stimulation by Oncostatin M.

The effects of genistein and vanadate on Oncostatin M-induced stimulation of tyrosine phosphorylation and LDLR expression are summarized in Table I below.

TABLE I

| EFFECT OF GENISTEIN AND VANADATE ON ONCOSTATIN M-INDUCED TYROSINE PHOSPHORYLATION AND LDLR EXPRESSION | | |
|---|---|---|
| | TYROSINE PHOSHORYLATION | LDL UPTAKE % STIMULATION |
| ONCOSTATIN M | + + | 75 |
| GENISTEIN | - | -10 |
| GENISTEIN + ONCOSTATIN M | + | 35 |
| VANADATE | + + + | 6 |
| VANADATE + ONCOSTATIN M | + + + + + | 125 |

6.2.5. EFFECT OF ONCOSTATIN M ON PHOSPHOLIPID METABOLISM

The Oncostatin M stimulation of tyrosine phosphorylation and diacylglycerol suggested that the factor might be causing the degradation of phospholipids. To study this possibility, the effect of Oncostatin M on HepG2 phospholipids was investigated. Oncostatin M increased radiolabel incorporation into phosphatidylcholine by 52% within 15 min (Figure 10). The incorporation remained elevated through 30 min. The factor also stimulated a 30% increase in radiolabeled polyphosphoinositides in the same time period (Figure 10).

The present invention is not to be limited by the embodiments disclosed herein which are intended as single illustrations of one aspect of the invention and any which are functionally equivalent are within the scope of the invention. Indeed, various modifications of the invention, in addition to those shown and described herein, will become apparent to those skilled in the art from the foregoing description. Such modifications are intended to fall within the scope of the appended claims.

**Claims**

1. The use of oncostatin M for preparing a pharmaceutical composition for stimulating the expression of low density lipoprotein receptors in hepatocytes.

2. The use of oncostatin M for preparing a pharmaceutical composition for enhancing the capacity of a hepatocyte to remove low density lipoprotein from the extracellular environment of the hepatocyte.

3. The use of oncostatin h for preparing a pharmaceutical composition for increasing the number of low density lipoprotein receptors expressed in the liver.

4. The use of oncostatin M for preparing a pharmaceutical composition for enhancing the capacity of the liver to remove plasma low density lipoprotein.

5. The use of oncostatin M in an amount effective at enhancing the capacity of an individual's liver for preparing a pharmaceutical composition for removing plasma low density lipoprotein.

6. The use of oncostatin M in an amount effective at reducing the level of circulating low density lipoprotein for preparing a pharmaceutical composition for treating atherosclerosis in an individual.

7. The use of oncostatin M in an amount effective at reducing the level of circulating low density lipoprotein for preparing a pharmaceutical composition for treating hypercholesterolemia in an individual.

8. The use according to claim 1, 2, 3, 4, 5, 6, or 7 in which the oncostatin M is covalently coupled to an antibody molecule that defines a cellular antigen, a hormone, a growth factor, or a cytokine.

9. The use according to claim 1, 2, 3, 4, 5, 6, or 7 in which oncostatin M is encapsulated in a liposome or a microcapsule.

**10.** A composition comprising oncostatin M covalently coupled to an antibody molecule that defines a cellular antigen, a hormone, a growth factor, or a cytokine.

**11.** A composition comprising oncostatin M encapsulated in a liposome or a microcapsule.

**12.** A process for preparing a composition as defined in any one of claims 1 to 11 which comprises providing oncostatin M in a form suitable for administration, optionally incorporated into a pharmaceutically acceptable carrier or diluent.

# *FIG. 1*

FIG. 2

# FIG.3

# FIG. 4

# F I G. 5

# *FIG. 6*

EP 0 485 961 A1

# FIG. 7

H 2981    HEPG2

Cold OM    — +    — +

Kd
200—

97—

68—

43—

25—

20

# FIG. 8

FIG. 9A

EFFECT OF ONCO M ON TYROSINE PHOSPHORYLATION: TIME DEPENDENCE

INHIBITION BY GENISTEIN

*FIG. 9B*

MOLECULAR WEIGHT

205 –

108 –

80 –

1     2     3     4

# FIG. 10

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CIRCULATION SUPPLEMENT vol. 82, no. 4, NEW YORK page 326; ROBERT I. GROVE ET AL: 'Oncostatin M upregulates LDL receptors by a sterol synthesis-independant mechanism' abstract  1294 *abstract* | 1-7,12 | A61K37/02 |
| Y | *abstract* | 8-9 | |
| X | WO-A-9 012 585 (ONCOGEN LIMITED PARTNERSHIP) * claims 41-46 * | 10-12 | |
| Y | * claims 41-46 * | 8-9 | |
| X,P | JOURNAL OF BIOLOGICAL CHEMISTRY. vol. 266, no. 27, BALTIMORE US ROBERT I. GROVE: 'Oncostatin m upregulates low density lipoproteins receptors in HePG2 cells by a novel mechanism' page 18194-18199 * the whole document * | 1-7,12 | |
| A,D | JOURNAL OF BIOLOGICAL CHEMISTRY. vol. 264, no. 8, BALTIMORE US pages 4282 - 4289; PETER S. LINSLEY ET AL: 'Identification and characterization of cellular receptors for the growth regulator, Oncostatin M' * the whole document * | 1-7 | TECHNICAL FIELDS SEARCHED (Int. Cl.5)<br><br>A61K<br>C07K |

The present search report has been drawn up for all claims

| Place of search | Date of completion of the search | Examiner |
|---|---|---|
| THE HAGUE | 11 FEBRUARY 1992 | FERNANDEZ Y BRA F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................................................

& : member of the same patent family, corresponding document

EPO FORM 1503 03.82 (P0401)